(19) Europäisches Patentamt
European Patent Office
Office européen des brevets

(11) Publication number: 0 051 946
A1

(12) EUROPEAN PATENT APPLICATION

(21) Application number: 81304985.5

(22) Date of filing: 22.10.81

(51) Int. Cl.³: C 07 D 323/00, C 07 F 9/50, C 07 F 15/00, C 07 F 1/00

(30) Priority: 07.11.80 GB 8035801

(43) Date of publication of application: 19.05.82 Bulletin 82/20

(84) Designated Contracting States: BE CH DE FR GB IT LI NL

(71) Applicant: IMPERIAL CHEMICAL INDUSTRIES PLC, Imperial Chemical House Millbank, London SW1P 3JF (GB)

(72) Inventor: Colquhoun, Howard Matthew, 25 Beeston Drive, Knutsford Cheshire (GB)
Inventor: Lewis, David Frank, 23 Lamswood Park, Hartford Northwich (GB)
Inventor: Stoddart, James Fraser, 23 Rosamond Drive, Bradway Sheffield (GB)

(74) Representative: Stephenson, Kenneth et al, Imperial Chemical Industries PLC Legal Department: Patents Thames House North Millbank, London SW1P 4QG (GB)

(54) **Metal complexes.**

(57) Adducts of macrocyclic ligands, especially crown ethers, with transition metal complexes in which ammonia or a primary amine (optionally with one or more other ligands) is co-ordinated to the transition metal.

The differing solubilities of the adducts provide a method of separating transition metal complexes.

EP 0 051 946 A1

Metal Complexes

The present invention relates to metal complexes and in particular to adducts of transition metal complexes with macrocyclic ligands.

According to the present invention we provide compounds having the general formula:

$$\left\{[ML_aL^1_b]^{n+}\right\}_x[L^2]_y[X]_z^{p-}$$

wherein M is a transition metal,

L is a ligand co-ordinated to said transition metal M selected from ammonia and primary amines,

$L^1$ is a ligand, other than ammonia or a primary amine co-ordinated to transition metal M,

$L^2$ is a macrocyclic ligand containing two or more electron donor atoms which are hydrogen bonded to acidic hydrogen atoms of ligand L or of ligands L and $L^1$,

X is an anion, when n is equal to or greater than 1,
n is equal to 0, 1, 2 or 3,
a is an integer from 1 up to the value required to satisfy the co-ordination requirements of metal M,
b is an integer from 0 up to the value required to satisfy the co-ordination requirements of M other than provided by the ligand(s) L,
x and y, which may be equal to one another, are positive integers,
p is 1, 2 or 3, and
z is equal to xn/p.

By a macrocyclic ligand we mean a ligand containing rings which have 12 or more atoms.

By a transition metal M we mean a metal which, in any one of its commonly occuring oxidation states, has a partly filled d shell. As described in "Advanced Inorganic Chemistry" by F A Cotton and G Wilkinson Chapter 10, 4th Edition, Interscience Publishers, 1980 such transition metal elements include a first series consisting of scandium, titanium, vanadium, chromium, manganese, iron, cobalt, nickel and copper; a second series consisting of zirconium, niobium, molybdenum, technetium, ruthenium, rhodium, palladium and silver; and a third series consisting of hafnium, tantalum, tungsten, rhenium, osmium, iridium, platinum and gold.

Suitably, the transition metal is iron (II and III), cobalt (II), nickel (II), copper (II), ruthenium (II), palladium (II), platinum (II) and iridium (I).

Suitable ligands L include ammonia; mono-amines e.g. alkylamines such as ethylamine and polyamines e.g. ethylene diamine and diethylene triamine.

Suitable ligands $L^1$, which may optionally contain acidic hydrogen atoms, include halide, e.g. chloride, carbonyl; dinitrogen; alkylphosphine; arylphosphine;

cyclopentadienyl; alkene; oxo; hydride; water; acetonitrile; monosubstituted acetonitriles, e.g. phenyl acetonitrile; and alkyl carbenes, e.g. methyl methoxy carbene.

Suitable anions $X^-$ include halide, e.g. chloride; nitrate; sulphate; carbonate; tetrafluoroborate $(BF_4)^-$; hexahalophosphate, e.g. hexafluorophosphate $(PF_6)^-$; hexahaloantimonate, e.g. hexachloroantimonate $(SbCl_6)^-$; tetraphenylborate $B(Ph)_4^-$; and hexahalosilicate, e.g. hexafluorosilicate $(SiF_6)^{2-}$.

Typical transition metal complexes $[ML_aL^1_b]^{n+}$ constituting a part of the compounds of the invention include $[Cr(NH_3)_6]^{3+}$, $[Cr(NH_3)_3Cl_3]$, $[Cr(NH_3)_5OCr(NH_3)_5]^{4+}$ $[Fe(ethylenediamine)_3]^{2+}$, $[Co(NH_3)_6]^{3+}$, $[Co(NH_3)_5Cl]^{2+}$ $[Co(NH_3)_3Cl_3]$, $[Co(ethylenediamine)_2(H_2O)_2]^{3+}$, $[Ni(NH_3)_6]^{2+}$ $[Cu(ethylenediamine)_2]^+$, $[Cu(NH_3)_4H_2O]^{2+}$, $[Ru(NH_3)_5N_2]^{2+}$ $[Ru(NH_3)_5NO]^{3+}$, $[Os(NH_3)_4N_2]^{2+}$, $[Rh(NH_3)_5Cl]^{2+}$ $[Rh(ethylenediamine)_2Cl_2]^+$, $[Rh(NH_3)_5H]^{2+}$ $[Ir(NH_3)_6]^{3+}$ $[Pd(NH_3)_4]^{2+}$, $[Pd(ethylenediamine)_2]^{2+}$, $[Pt(PMe_3)Cl_2NH_3]$, $[Au(ethylenediamine)_2]^{3+}$ and $[Ag(NH_3)_2]^+$.

The ligand $L^2$ typically includes macrocyclic compounds containing oxygen, nitrogen or sulphur as the electron donor atoms, as well as macrocyclic compounds containing nitrogen-oxygen, sulphur-nitrogen, sulphur-oxygen and nitrogen-sulphur-oxygen donor atoms. Such macrocyclic compounds include cyclic polyethers (so-called "crown ethers"), cyclic polyamines and cyclic polythioethers, as well as the mixed donor macrocyclic compounds containing oxygen and/or nitrogen and/or sulphur. An extensive review of such compounds is given, for example, by J. J. Christensen et al. in "Chemical Reviews" 1974 , 74, (3), 351-384.

Preferably, the macrocyclic ligand $L^2$ is a cyclic polyether (or crown ether) which may be achiral or chiral. Especially preferred ligands include those having the following structures, the nomenclature used being that

devised by Pedersen, J. Amer. Chem. Soc., 89, 7017 (1967) for macrocyclic polyethers, whereby each name consists of, in order: (1) the number and kind of hydrocarbon rings, (2) the total number of atoms in the polyether ring, (3) the class name, "crown", and (4) the number of oxygen atoms in the polyether ring:

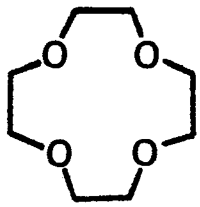

12-Crown-4

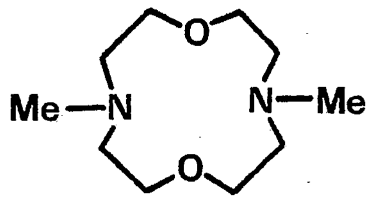

N,N'-dimethyl-
diaza-12-Crown-4

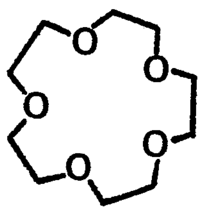

15-Crown-5

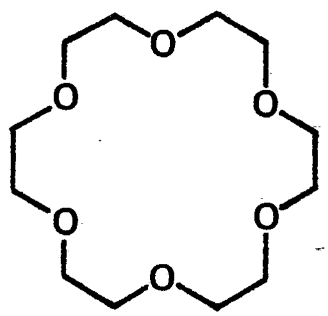

18-Crown-6

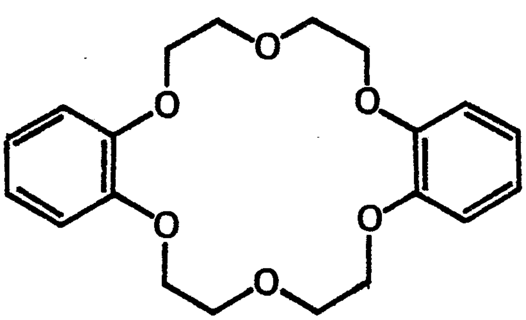

Dibenzo-18-Crown-6

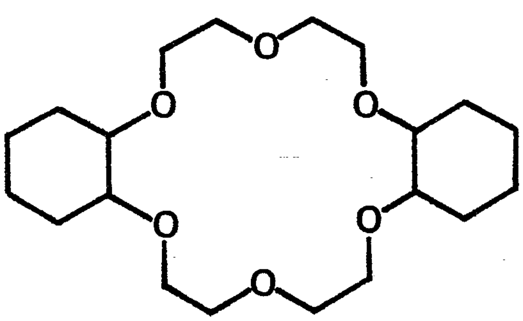

Dicyclohexyl-18-Crown-6

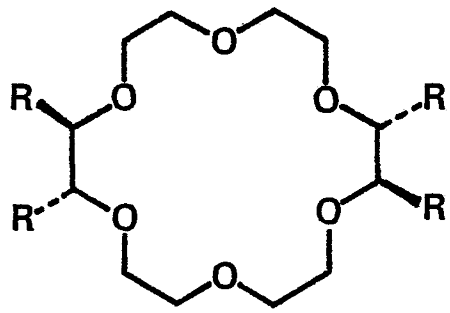

Bisthreitolo-18-Crown-6

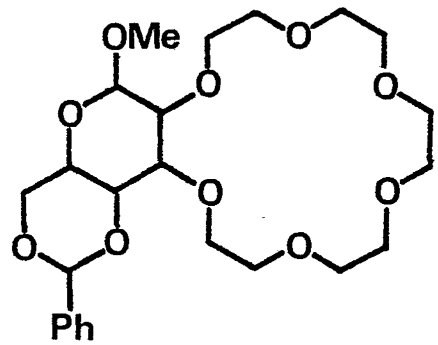

Glycosido-18-Crown-6

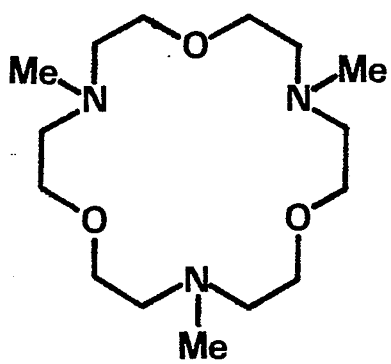

N,N',N''-trimethyl-
triaza-18-Crown-6

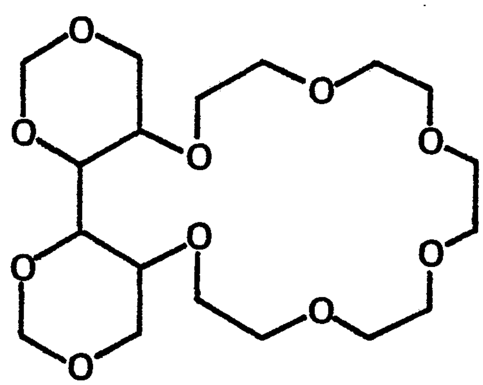

Dimethylenemannitolo-20-Crown-6

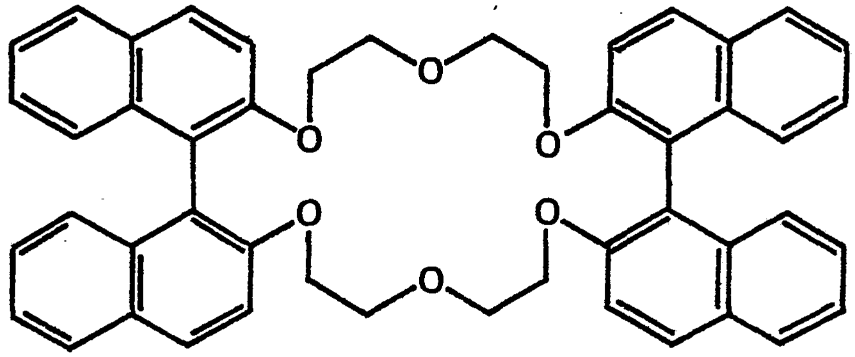

Bisbinaphtho-22-Crown-6

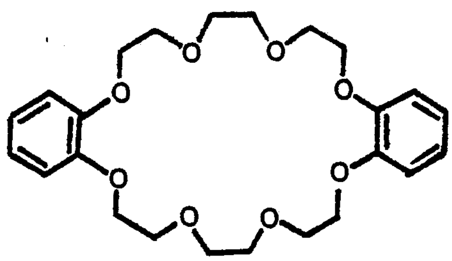

Dibenzo-24-Crown-8

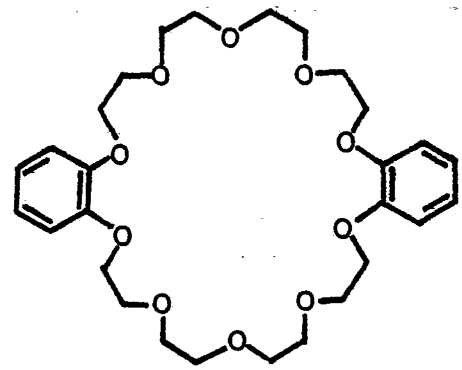

Dibenzo-30-Crown-10

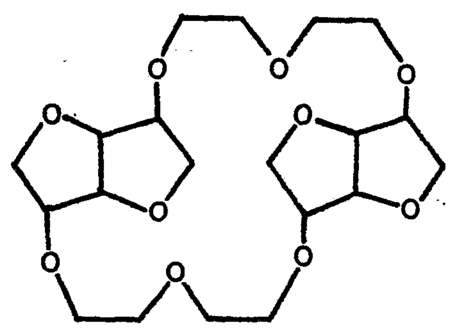

Bisdianhydromannitolo-30-Crown-10

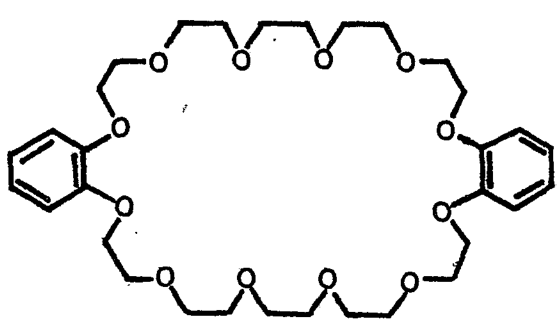

Dibenzo-36-Crown-12

The compounds of the invention are typically prepared by mixing aqueous or non-aqueous solutions of the transition metal complex and the macrocyclic ligand so as to form the desired adduct of the transition metal complex with the macrocyclic ligand, which adduct may precipitate directly from the reaction mixture or be precipitated by the further addition of a suitable solvent.

The compounds of the invention include not only those adducts of transition metal complexes with macrocyclic ligands which are stable in the solid state, but also adducts which are detectable, e.g. by spectroscopy, in aqueous and/or non-aqueous solution.

Specific novel compounds according to the invention include compounds having the following formulae:

$\{[(PMe_3)PtCl_2NH_3]_2.18\text{-crown-6}\}$ I

$\{[(PEt_3)PtCl_2NH_3]_2.18\text{-crown-6}\}$ II

$[(PMe_3)PtCl_2NH_3.\text{dibenzo-18-crown-6}]$ III

$[(PEt_3)PtCl_2NH_3.\text{dibenzo-8-crown-6}]$ IV

$\{[(C_2H_4)PtCl_2.NH_3]_3(18\text{-crown-6})_2\}$ V

$[(Bipy)Pt(NH_3)_2.18\text{-crown-6}]^{2+}[PF_6]^-_2$ VI

$[(Bipy)Pt(NH_3)_2.\text{dibenzo-30-crown-10}]^{2+}[PF_6]^-_2H_2O$ VII

$[\underline{trans}\text{-}PtCl_2(NH_3)_2.18\text{-crown-6}]_n$ VIII

$[Pt(NH_2C_2H_4NH_2)_2.18\text{-crown-6}]^{2+}[PF_6]^-_2$ IX

$[Cu(NH_3)_4H_2O.18\text{-crown-}6]^{2+}[PF_6]_2^-$ ... X

$\{[Co(NH_3)_6]_2\ (18\text{-crown-}6)_3\}^{6+}[PF_6]_6^-$ XI

The compounds according to the invention facilitate the separation of transition metal complex ions in solution. Thus the separation of a particular transition metal complex ion from one or more other transition metal complex ions in solution may be effected by forming an insoluble adduct between the complex ion to be separated and a macrocyclic compound. The insoluble adduct is then separated from the solution and the adduct is treated to recover both the desired transition metal ions and the macrocyclic compound. The latter can then be recycled to react with fresh solutions containing complex ions of the desired transition metal.

A particular application of the compounds of the invention involves the separation of a copper ammine complex from other metallic (especially cobalt) ammine complexes in solutions obtained, for example, from the ammonia leaching of carbonaceous ore deposits and ammoniacal-oxidation leaching of copper sulphide concentrates. The ammonia leach solution typically comprises the copper ammine complex $[Cu(NH_3)_4(H_2O)]^{2+}$ and the cobalt ammine complex $[Co(NH_3)_6]^{3+}$ and in accordance with a further aspect of the present invention, the said metal complexes are treated with a macrocyclic compound, preferably a crown-ether, and especially in the presence of an anion such as a hexahalophosphate ion e.g. $[PF_6]^-$, to form an insoluble adduct of the copper ammine complex with the macrocyclic compound which is precipitated and a soluble adduct of the cobalt ammine complex and/or other transition metal ammine complexes, if present) which remains in solution. The precipitated adduct is separated from the

solution and acidified to liberate copper ions and the macrocyclic compound. In a preferred separation process, the macrocyclic compound is a crown-ether, and in an especially preferred process, the regenerated macrocyclic compound after acidification is an ammonium crown-ether/hexahalophosphate complex, e.g. $[NH_4.18\text{-crown-}6]^+[PF_6]^-$. Such a complex is soluble in organic solvents, e.g. methylene dichloride, thereby enabling the aqueous copper ions to be separated from the crown-ether. After removal of organic solvent, e.g. by evaporation, the ammonium crown-ether/hexahalophosphate complex, if desired, may be recycled and used in place of the crown-ether to separate copper from cobalt in fresh supplies of ammonia leach solutions.

The compounds of the invention may also be used to separate precious metal complexes (e.g. complexes of the platinum group metals, silver and gold).

The invention is illustrated by the following Examples.

Example 1

$[(PEt_3)PtCl_2NH_3$.Dibenzo-18-crown-6] (IV) and Related Compounds

$(PEt_3)PtCl_2NH_3$ (0.27 g, 0.67 mmol) and dibenzo-18-crown-6 (0.25 g 0.67 mmol) were dissolved in $CH_2Cl_2$ (5 $cm^3$) and the solution was filtered. Hexane was added dropwise with stirring until a faint permanent precipitate was observed. After standing for 30 minutes at room temperature the adduct had crystallised as long fibrous needles of a dichloromethane solvate which were filtered off, washed with hexane, and dried. The yield was 0.40 g (77%).

The following compounds were similarly prepared:

$\{[(PMe_3)PtCl_2NH_3]_2.18\text{-crown-}6\}$ I

$\{[(PEt_3)PtCl_2NH_3]_2.18\text{-crown-}6\}$ II

$[(PMe_3)PtCl_2NH_3.\text{dibenzo-}18\text{-crown-}6]$ III

$\{[(C_2H_4)PtCl_2NH_3]_3.(18\text{-crown-}6)_2\}$ V

The compounds were characterised as follows:

| | Mpt (°C) | Elemental Analysis Calculated values in brackets C% | H% | N% |
|---|---|---|---|---|
| (I) | 176 | 22.7(22.0) | 5.4(4.9) | 2.9(2.8) |
| (II) | 169 | 27.5(27.0) | 6.2(5.6) | 2.6(2.6) |
| (III) | 177 | 38.7(38.7) | 5.0(5.0) | 1.8(2.0) |
| (IV) | 129 | 38.5(38.3) | 5.2(5.2) | 1.9(1.7) |
| (V) | 162 | 24.3(24.6) | 4.7(4.7) | 2.8(2.9) |

Infra-red spectra ($cm^{-1}$ Nujol Mull)

| | |
|---|---|
| I | 3340 m, 3315 m, 3270 w, 3200 m (νNH), 1640 m, 1630 m (δNH), 1110 s (νCO) |
| II | 3330 s, 3265 m, 3200 m (νNH), 1635 m (δNH), 1115 s (νCO) |
| III | 3340 m, 3280 m, 3190 w (νNH), 1600 m (δ NH), 1140 s (νCO) |
| IV | 3320 m, 3260 m, 3170 w (νNH), 1600 m (δ NH) 1130 s (νCO) |
| V | 3280 s, 3190 s (νNH), 1628 m, br (δNH), 1105 (νCO) |

Proton n.m.r. spectra

I $^1H$ N.m.r data for {[(PMe$_3$)PtCl$_2$NH$_3$]$_2$.18-crown-6} in $CD_2Cl_2$ : δ 1.49 (18H, d with $^{195}Pt$ satellites, $\underline{J}_{PH}$ = 12 Hz, 2x $PMe_3$), 3.00 (6H,bs,2 x $NH_3$), and 3.61 (24H, s, $OCH_2CH_2O$ protons)

II $^1H$ N.m.r data for{[(PEt$_3$)PtCl$_2$NH$_3$]$_2$.18-crown-6} in $CD_2Cl_2$ : δ 1.09-1.30 (18H, m, 2 x $(PCH_2C\underline{H}_3)_3$), 1.72-2.00 (12H, m, 2 x $(PC\underline{H}_2CH_3)_3$), 3.02 (6H, bs, 2 x $NH_3$), and 3.65 (24H, s, $OCH_2CH_2O$ protons)

III $^1H$ N.m.r data for {(PMe$_3$)PtCl$_2$NH$_3$.dibenzo-18-crown-6}in $CD_2Cl_2$ : δ 1.44 (9H, d with $^{195}Pt$ satellites, $\underline{J}_{PH}$ = 12Hz, $PMe_3$), 3.00 (3H, bs, $NH_3$), 3.94-4.22 (16H, $A_2B_2$ system for $OCH_2CH_2O$ protons), and 6.87 (8H, bs aromatic protons)

V $^1H$ N.m.r data for 3:2 complex between [($C_2H_4$) PtCl$_2$NH$_3$] and 18-crown-6 in $CD_2Cl_2$: δ 3.63 (s, $OCH_2CH_2O$ protons), and 4.49 (s with $^{195}Pt$ satellites, $\underline{J}_{PtH}$ = 60 Hz, olefinic protons)

Example 2

[(Bipy)Pt(NH$_3$)$_2$.18-crown-6]$^{2+}$[PF$_6$]$^-_2$ and [(Bipy)Pt(NH$_3$)$_2$ dibenzo-30-crown-10]$^{2+}$ [PF$_6$]$^-_2$H$_2$O

(Bipy) = 2,2' bipyridyl

[(Bipy)Pt(NH$_3$)$_2$]$^{2+}$[PF$_6$]$^-_2$ (0.33g, 0.43 mmol) and 18-crown-6 (0.23g, 0.87 mmol) were dissolved in $CH_2Cl_2$ (30 cm$^3$) and $Et_2O$ was added dropwise until the first trace of permanent cloudiness was evident. The solution was allowed to stand overnight and the resulting yellow crystals were filtered off, washed with $Et_2O$, and dried. The yield was 0.27g (61%).

The corresponding adduct with dibenzo-30-crown-10 was prepared similarly, but analysis and i.r. spectroscopy indicated the presence of a mole of water in this adduct.

The compounds (VI) and (VII) were characterised as follows:

| | Mpt($^{o}$C) | Elemental Analyses Calculated values in brackets | | |
|---|---|---|---|---|
| | | C% | H% | N% |
| (VI) | 231 | 28.2(28.1) | 4.0(4.0) | 5.7(6.0) |
| (VII) | 191 | 37.4(37.1) | 4.1(4.5) | 4.3(4.5) |

Infra-red spectra (cm$^{-1}$ Nujol Mull)

VI 3200 m, br (νNH), 1620 m (δNH), 1105 s (νCO) 820 vs (νPF)

VII 3660 m, 3590 (νOH), 3200 m, br, (νNH), 1630 m, 1610 m, 1595 m (δOH/NH), 1100 s, br (νCO) 820 vs (νPF)

Proton n.m.r. spectra

VI $^{1}$H N.m.r data for $[(Bipy)Pt(NH_3)_2.18\text{-crown-}6]^{2+}[PF_6]_2^-$ in $CD_2Cl_2$ : δ 3.74 (24H, s, $OCH_2CH_2O$, protons), 4.77 (6H, bs, 2 x $NH_3$) and 7.68-8.0 (8H, t, m, and d, aromatic protons)

VII $^{1}$H N.m.r data for $[(Bipy)Pt(NH_3)_2.\text{dibenzo-}30\text{-crown-}10]^{2+}[PF_6]_2^-$ in $CD_2Cl_2$ : δ 3.65-3.96 (32H, 2 x s and $A_2B_2$ system, $OCH_2CH_2O$ protons) 4.62 (6H, bs, 2 x $NH_3$), 6.36 (8H, s, benzo aromatic protons) and 7.64-8.42 (8H, m, t and d, pyridyl aromatic protons)

Example 3

$[\textit{trans}\text{-}PtCl_2(NH_3)_2.18\text{-crown-}6]_n$ (VII)

Trans-$PtCl_2(NH_3)_2$ (0.30g, 1.0 mmol) was dissolved in dimethylformamide (30 cm$^3$) at 40$^{o}$C and 18-crown-6 (0.26g, 1.0 mmol) in d.m.f. (10 cm$^3$) was added dropwise with stirring. A precipitate formed immediately, but dissolved on boiling. Slow cooling of this solution gave pale yellow needles of the adduct, which were filtered off, washed with cold d.m.f. and $Et_2O$, and dried under vacuum. The yield was 0.53g (95%).

The compound (VII) was characterised as follows:

| | MPt (°C) | Elemental Analysis Calculated values in Brackets | | |
|---|---|---|---|---|
| | | C% | H% | N% |
| VII | 225 | 25.7(25.5) | 5.2(5.3) | 4.9(5.0) |

Infra-red spectrum ($cm^{-1}$ Nujol Mull)

VII 3320 s, 3215 s (νNH), 1630 m (δNH), 1115 (νCO)

Example 4

$[Pt(NH_2C_2H_4NH_2)_2 \cdot 18\text{-crown-}6]^{2+}[PF_6]_2^-$ (IX)

$[Pt(NH_2C_2H_4NH_2)]^{2+}[PF_6]_2^-$ (0.24 g, 0.40 mmol) was dissolved in 5 $cm^3$ of water, and 18-crown-6 (0.20 g, 0.76 mmol) in 2 $cm^3$ of water was added. The solution was cooled to 5°C for 1 hour and the resulting crystals were filtered off, washed with a little ice-water and $Et_2O$, and dried under vacuum. The yield was 0.17g, 49%. The compound was characterised as follows:

| | MPt (°C) | Elemental Analysis Calculated values in Brackets | | |
|---|---|---|---|---|
| | | C% | H% | N% |
| IX | 201 | 21.9(22.1) | 4.6(4.6) | 6.4(6.4) |

Infra-red spectrum ($cm^{-1}$ Nujol Mull)

IX 3305 s, 3220 m, 3140 m (νNH) 1600 m (δNH) 1115 s, (νCO), 820 vs (νPF)

IX $^1H$ N.m.r. data for $[Pt(NH_2C_2H_4NH_2)_2 \cdot 18\text{-crown-}6]^{2+}[PF_6]_2^-$ in $D_2O$ : δ 2.64 (8H, s, $NCH_2CH_2N$ protons) and 3.67 (24H, s, $OCH_2CH_2O$ protons).

Example 5

$[Cu(NH_3)_4H_2O.18\text{-crown-}6]^{2+}[PF_6]_2^-$ (X)

$[Cu(NH_3)_4H_2O]^{2+}[PF_6]_2^-$ (0.95g 2.16 mmol) was dissolved in the minimum volume of 3M aqueous ammonia, and 18-crown-6 (0.57g 2.16 mmol) in 2 $cm^3$ of water was added. The solution was allowed to stand at room temperature for 3 hours, and the resulting deep blue crystals were filtered off, washed with a little ice-water and $EtO_2$, and dried on the filter. The yield was 0.52g, 34%.

The compound (X) was characterised as follows:

| | MPt (°C) | Elemental Analysis Calculated values in brackets | | |
|---|---|---|---|---|
| | | C% | H% | N% |
| X | 183 | 20.9(20.5) | 5.4(5.4) | 8.1(8.1) |

Infra-red spectrum ($cm^{-1}$ Nujol Mull)

X 3640 w, 3555 w (νOH), 3355 s, 3270 m, 3205 m (νNH), 1635 m (δNH/OH), 1100 s (νCO), 830 s (νPF)

Example 6

$\{[(Co(NH_3)_6]_2\ (18\text{-crown-}6)_3\}^{6+}[PF_6]_6^-$ (XI)

$[Co(NH_3)_6]^{3+}[PF_6]_3^-$ (0.60g 1.0 mmol) was dissolved in 20 $cm^3$ of water and 18-crown-6 (0.52g 2.0 mmol) in 5 $cm^3$ of water was added. On standing at room temperature for 1 hour, orange crystals formed, and these were filtered off, washed with a little water and $Et_2O$, and dried. The yield was 0.54g (54%).

The compound (XI) was characterised as follows:

| | MPt (°C) | Elemental Analysis Calculated values in brackets | | |
|---|---|---|---|---|
| | | C% | H% | N% |
| XI | > 250 | 21.4(21.8) | 4.6(5.4) | 7.7(8.5) |

Infra-red spectrum ($cm^{-1}$ Nujol Mull)

XI 3350 vs, br (νNH), 1630 m, br (δNH), 1100 s (νCO) 830 vs (νPF)

Example 7

Separation of $[(Cu(NH_3)_4H_2O]^{2+}[PF_6]_2^-$ from $[Co(NH_{3}\ _6]^{3+}[PF_6]_3^-$ by reaction with 18-crown-6 or $[NH_4\cdot$18-crown-6$]^+[PF]_6^-$

$[Cu(NH_3)_4H_2O]^{2+}[PF_6]_2^-$ (0.84g 2.0 mmol) and $[Co(NH_3)_6]^{3+}[PF_6]_3^-$ (1.20g, 2.0 mmol) were dissolved in 3M aqueous ammonia (40 $cm^3$) and this solution was treated with a solution of 18-crown-6 (0.57g, 2.16 mmol) in water (5 $cm^3$). After cooling this mixture in ice for 30 minutes, the resulting blue crystals of $[Cu(NH_3)_4H_2O$.18-crown-6$]^{2+}[PF_6]_2^-$ (X) were filtered off, washed with $Et_2O$, and dried. The yield was 1.27g (93%). This material was shaken with 1M aqueous HCl and the aqueous suspension extracted with 2 x 150 $cm^3$ of $CH_2Cl_2$. The organic layer was separated, dried over 3A molecular sieve, and evaporated to dryness, giving 0.52g of $[NH_4\cdot$18-crown-6$]^+[PF_6]^-$ (86%). The latter may be used in place of free 18-crown-6 to separate $Cu^{3+}$ from $Co^{3+}$.

Thus, on shaking solid $[NH_4\cdot$18-crown-6$]^+[PF_6]^-$ (0.95g, 2.23 mmol) with 40 $cm^3$ of 3M aqueous ammonia containing 0.84g (2.0 mmol) of $[Cu(NH_3)_4H_2O]^{2+}[PF_6]_2^-$ and 1.20g (2.0 mmol) of $[Co(NH_3)_6]^{3+}[PF_6]_3^-$ for 1 hour, followed by cooling in ice for 30 minutes, a 93% yield of blue crystalline $[Cu(NH_3)_4H_2O$.18-crown-6$]^{2+}[PF_6]_2^-$ (X) was obtained.

Example 8

Separation of a platinum complex from a rhodium complex

A solution containing $[Pt(NH_3)_4]^{2+}[PF_6]_2^-$ (0.28g) and $[Rh(NH_3)_5Cl]^{2+}[PF_6]_2^-$ (0.29g) in 30 $cm^3$ of water was treated with a solution of 18-crown-6 in 2 $cm^3$ of water. After ½ hour, white crystals of an adduct between $[Pt(NH_3)_4]^{2+}[PF_6]_2^-$ and 18-crown-6 had separated, and were filtered off (yield 0.25g) leaving the rhodium complex in solution.

Example 9

Removal of *trans* $PtCl_2(NH_3)_2$ from *cis* $PtCl_2(NH_3)_2$

A qualitative experiment showed that addition of 18-crown-6 to a dimethylformamide solution of *cis* $PtCl_2(NH_3)_2$ produced no precipitated adduct. Since, as shown in Example 3, the *trans* isomer gives an immediate precipitate of the polymeric adduct [*trans* $PtCl_2(NH_3)_2$·18-crown-6]$_n$, the addition of 18-crown-6 to mixtures of these isomers affords a simple method of removing the *trans* isomer selectively.

What we claim is:

1. A compound of the formula:

$$\left\{[ML_aL^1_b]^{n+}\right\}_x [L^2]_y [X]^{p-}_z$$

wherein M is a transition metal,

L is a ligand co-ordinated to said transition metal M selected from ammonia and primary amines,

$L^1$ is a ligand other than ammonia or a primary amine, co-ordinated to transition metal M,

$L^2$ is a macrocyclic ligand containing two or more electron donor atoms which are hydrogen bonded to acidic hydrogen atoms of ligand L or of ligands L and $L^1$,

X is an anion, when n is equal to or greater than 1,

n is equal to 0, 1, 2 or 3,

a is an integer from 1 up to the value required to satisfy the co-ordination requirements of metal M,

b is an integer from 0 up to the value required to satisfy the co-ordination requirements of M other than provided by the ligand(s) L,

x and y, which may be equal to one another, are positive integers,

p is 1, 2 or 3, and

z is equal to xn/p.

2. A compound according to claim 1 wherein the transition metal is selected from iron (II and III), cobalt (II), nickel (II), copper (II), ruthenium (II),

palladium (II), platinum (II) and iridium (I).

3. A compound according to claim 1 or claim 2 wherein the macrocyclic ligand is a crown ether.

4. A compound according to claim 3 wherein the crown ether is selected from 18-crown-6, dibenzo-18-crown-6 and dibenzo-30-crown-10.

5. A method for the preparation of a compound as claimed in claim 1 which comprises mixing solutions containing a transition metal complex

$$\left\{[ML_aL^1_b]^{n+}\right\}_x [X]^{p-}_z$$

and a macrocyclic ligand $L^2$.

6. A method according to claim 5 wherein a solution containing at least two transition metal complexes is mixed with the macrocyclic ligand; the resulting adducts then being separated in accordance with their different solubilities.

7. A method according to claim 5 or claim 6 wherein the macrocyclic ligand is a crown ether.

K STEPHENSON
AGENT FOR THE APPLICANTS

0051946

Application number

European Patent Office

# EUROPEAN SEARCH REPORT

EP 81 30 4985

## DOCUMENTS CONSIDERED TO BE RELEVANT

| Category | Citation of document with indication, where appropriate, of relevant passages | Relevant to claim |
|---|---|---|
| | JOURNAL OF THE CHEMICAL SOCIETY, CHEMICAL COMMUNICATIONS, January 1980, LETCHWORTH (GB) G.R. NEWKOME et al.: "2,2'-Bipyridyl 'Crown Ethers'. Synthesis and X-Ray Crystal Structure of a Cobalt (II) Complex", pages 9-11 * the whole article * | 1-3 |
| | -- | |
| | JOURNAL OF THE CHEMICAL SOCIETY, DALTON TRANSACTIONS, December 1978, LETCHWORTH (GB). E.M. HYDE et al.: "Complexes of Platinum Metals with Crown Ethers containing Tertiary Phosphine-substituted Benzo Groups", pages 1696-1705 * page 1699, right-hand column to page 1703 * | 1-5,7 |
| | -- | |
| | JOURNAL OF THE CHEMICAL SOCIETY, DALTON TRANSACTIONS, October 1978, LETCHWORTH (GB). B.I. SHAW et al.: "Complexes of Palladium and Platinum with Substituted Benzo-(15-crown-5)‡ Ethers containing Nitrogen-donor Ligand Atoms", pages 1634-1638 * the whole article * | 1,3,5,7 |
| | -- | |
| | CHEMICAL ABSTRACTS, vol. 89, no. 12, 18th September, 1978, page 600, abstract 99126t, COLUMBUS, OHIO (US) M. YOSHIO et al.: "Analytical applications of crown ether. Extraction of cobalt thiocyanate ./. | |

CLASSIFICATION OF THE APPLICATION (Int. Cl. 3)

C 07 D 323/00
C 07 F 9/50
C 07 F 15/00
C 07 F 1/00

TECHNICAL FIELDS SEARCHED (Int.Cl. 3)

C 07 D 323/00
C 07 F 9/00
C 07 F 15/00
C 07 F 1/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 05.02.1982 | NUYTS |

European Patent Office

# EUROPEAN SEARCH REPORT

Application number
EP 81 30 4985



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | complex with ammonium-crown ether complex"<br>& Anal. Lett. 1978, A11(4),281-6<br><br>* the whole abstract *<br>--- | <br><br><br><br>1-4 | |
| A/D | JOURNAL OF THE AMERICAN CHEMICAL SOCIETY, vol. 89, no. 26, 20th December, 1967,<br>COLUMBUS, OHIO (US)<br>C.J. PEDERSON: "Cyclic Polyethers and Their Complexes with Metal Salts", pages 7017-7036<br><br>* page 7019 at the bottom to page 7024; table XIII; page 7028; pages 7031-7036 *<br>--- | <br><br><br><br><br><br>1-5,7 | TECHNICAL FIELDS SEARCHED (Int. Cl.3) |
| P | JOURNAL OF THE CHEMICAL SOCIETY, CHEMICAL COMMUNICATIONS, 1981,<br>LETCHWORTH (GB)<br>H.M. COLQUHOUN: "Second-sphere Co-ordination of Neutral and Cationic Transition Metal Complexes by Crown Ethers", pages 612 and 613<br><br>* the whole article *<br>--- | <br><br><br><br><br><br>1,3-5,7 | |
| P | JOURNAL OF THE CHEMICAL SOCIETY, CHEMICAL COMMUNICATIONS, August 1981,<br>LETCHWORTH (GB)<br>H.M. COLQUHOUN et al.: "The Binding of Neutral Platinum Complexes by Crown Ethers. X-Ray Crystal Structures of (trans-Pt $Cl_2(PMe_3)NH_3$-dibenzo-18-crown-6) and ({trans-$PtCl_2(PMe_3)NH_3$}$_2$.18-crown-6)", pages 847-849<br><br>* the whole article *<br>---<br>./. | <br><br><br><br><br><br><br><br><br>1-5,7 | |

European Patent Office

# EUROPEAN SEARCH REPORT

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| P | JOURNAL OF THE CHEMICAL SOCIETY, CHEMICAL COMMUNICATIONS, August 1981, LETCHWORTH (GB) H.M. COLQUHOUN et al.: "Formation and X-Ray Crystal Structure of $(Pt(H_2NCH_2CH_2NH_2)_2.18\text{-crown-}6)_n^{2+}(PF_6)_{2n}$. A Hydrogen Bonded Stepped-chain Copolymer", pages 851-852<br>* the whole article * | 1-5,7 | |
| | | | TECHNICAL FIELDS SEARCHED (Int. Cl.3) |
| P | JOURNAL OF THE CHEMICAL SOCIETY, CHEMICAL COMMUNICATIONS, August 1981, LETCHWORTH (GB) H.M. COLQUHOUN et al.: "Isolation and X-Ray Crystal Structure of $(Cu(NH_3)_4H_2O.18\text{-crown-}6)_n^{2+}(PF_6)_{2n}^{-}$ A Linear Face-to-face Hydrogen Bonded Chain Copolymer", pages 849-850<br>* the whole article * | 1-5,7 | |